**Europäisches Patentamt**

**European Patent Office**

**Office européen des brevets**

(19)

(11) Veröffentlichungsnummer: **0 165 542**

**A2**

(12) # EUROPÄISCHE PATENTANMELDUNG

(21) Anmeldenummer: **85107175.3**

(22) Anmeldetag: **11.06.85**

(51) Int. Cl.⁴: **A 61 M 5/31**

(30) Priorität: **16.06.84 DE 3422507**

(43) Veröffentlichungstag der Anmeldung:
**27.12.85 Patentblatt 85/52**

(84) Benannte Vertragsstaaten:
**AT BE CH FR GB IT LI NL SE**

(71) Anmelder: **Almo Erzeugnisse Erwin Busch GmbH**
**Grosse Allee 84**
**D-3548 Arolsen(DE)**

(72) Erfinder: **Busch, Jochen, Dipl.-Kaufm.**
**Georg Friedrichstrasse 12**
**D-3548 Arolsen(DE)**

(74) Vertreter: **Walther, Horst, Dipl.-Ing.**
**Wilhelmshöher Allee 275 Postfach 41 01 08**
**D-3500 Kassel(DE)**

(54) **Injektionsspritze, insbesondere Einmalinjektionsspritze.**

(57) Injektionsspritze, insbesondere eine Einmalinjektionsspritze, bestehend aus einem Spritzenzylinder sowie einem
an dem Zylinderboden angesetzten Ansatz, welcher die
Kanüle trägt, wobei der Ansatz (3) einen Füllkörper (5)
aufweist, der mit einem Durchlaß für die medikamentöse
Flüssigkeit versehen ist.

*Fig. 1*

EP 0 165 542 A2

# Dipl.-Ing. HORST WALTHER

Zugelassener Vertreter beim Europäischen Patentamt

# PATENTANWALT

## 0165542

Postscheckgiro-Kto 149359-602 Ffm.
Bankkonten in Kassel:
Raiffeisenbank 6573355 (BLZ 52060515)
Dresdner Bank 425498300 (BLZ 52080080)

W.-Germany
3500 Kassel-Wilh
Wilhelmshöher Allee 275
Postfach 410108
Telefon 0561/38714

Dipl.-Ing. H. Walther · 3500 Kassel · Wilhelmshöher Allee 275

Tag:

07. Juni 1984 W/Du

846/10592

Firma
ALMO Erzeugnisse
Erwin Busch GmbH

Große Allee 84

3548 Arolsen

---

Injektionsspritze, insbesondere Einmalinjektionsspritze

---

Die Erfindung betrifft eine Injektionsspritze,
insbesondere eine Einmalinjektionsspritze, bestehend aus einem Spritzenzylinder sowie einem
an dem Zylinderboden angesetzten Ansatz, welcher
die Kanüle trägt.

Es sind Injektionsspritzen, insbesondere sogenannte Einmalinjektionsspritzen aus Kunststoff bekannt,

- 2 -

0165542

- 2 -

bei denen am Zylinderboden ein konischer Ansatz
mit zentrischer Bohrung angebracht ist, auf den
die mit einem Kegelaufsatz versehene Kanüle geschoben wird.

Diese Ausbildung hat aber den Nachteil, daß nach
dem Spritzen einer medikamentösen Flüssigkeit,
immer noch ein gewisses Restvolumen verbleibt,
das sich in der relativ großen Bohrung des Ansatzes ansammelt. Dieses Restvolumen ist deshalb
nicht verwertbar, weil der im Spritzenzylinder
verschiebbare Kolben nur bis zu dem Zylinderboden
reicht und mithin alle Flüssigkeit, die sich in
Hohlräumen ansammelt, die an den Zylinderboden
anschließen, für die Verwertung verloren ist.

Der Erfindung liegt daher die Aufgabe zugrunde
eine Injektionsspritze zu schaffen, bei der kein
Restvolumen übrig bleibt, wenn man einmal von
dem geringen Kanülenvolumen absieht.

Dies wird erfindungsgemäß dadurch erreicht, daß
der Ansatz einen Füllkörper aufweist, der mit
einem Durchlaß für die medikamentöse Flüssigkeit
versehen ist. Nach der einen Ausführungsform
kann der Durchlaß aus einer Rille bestehen, die
am Umfang des Füllkörpers angebracht ist. Nach

- 3 -

0165542

- 3 -

einer anderen Ausführungsform kann der Füllkörper als Durchlaß mittig eine durchgehende Bohrung aufweisen.

Auf dem Umfang des Füllkörpers ist endseitig eine vorzugsweise als Bund ausgebildete Verdickung angebracht, die die ortsfeste Lage des Füllkörpers im Ansatz sicherstellt. Die Länge des Füllkörpers entspricht der Bohrungslänge des Ansatzes.

Durch den im Ansatz befindlichen Füllkörper wird erreicht, daß wegen des bündigen Abschließens des Füllkörpers mit dem Spritzenzylinderboden in der Endstellung des Kolbens kein Restvolumen mehr im Ansatz vorhanden ist. Der Vorteil dieser Ausführungsform besteht neben der nahezu völligen Restvolumenfreiheit darin, daß die Konstruktion des Spritzenzylinders beibehalten werden kann und somit auf dem Markt befindliche Spritzen mit diesem Füllkörper nachgerüstet werden können.

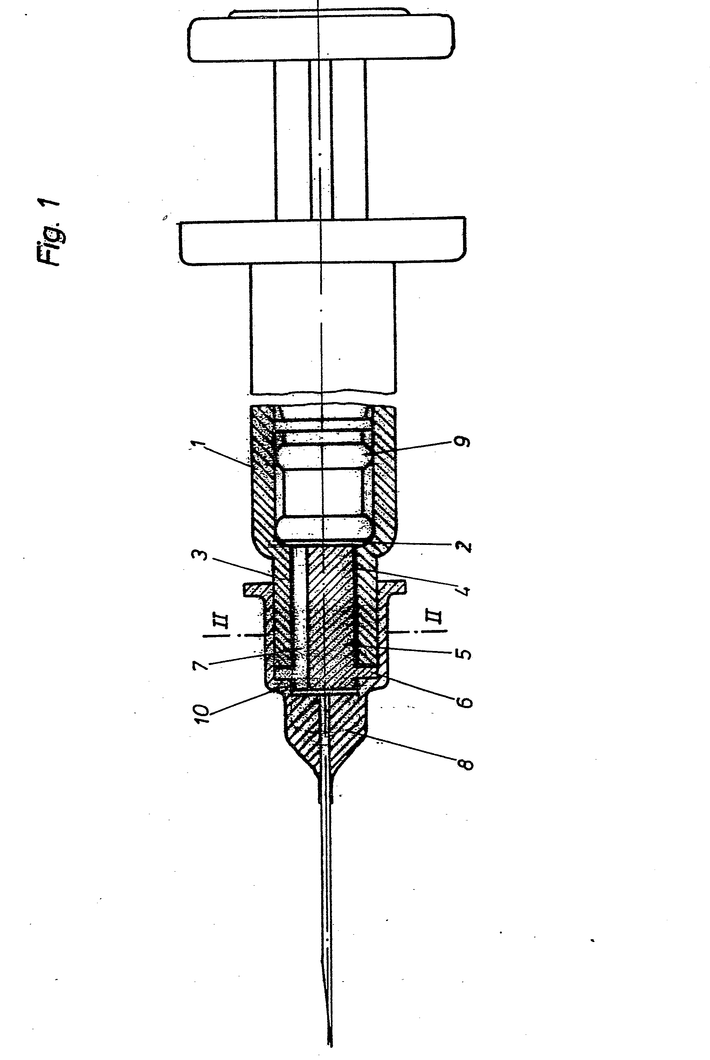

Fig. 1 zeigt eine Ansicht der Spritze;

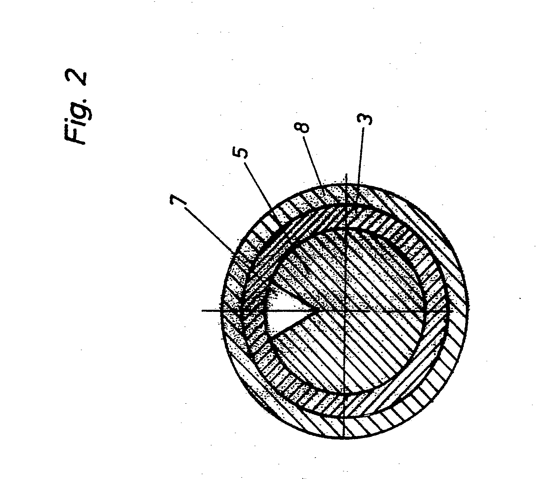

Fig. 2 einen Schnitt durch die Spritze gemäß der Linie II-II.

Mit 1 ist der Spritzenzylinder bezeichnet, der den Zylinderboden 2 aufweist. Am Zylinderboden 2

- 4 -

- 4 -

ist ein Ansatz 3 angesetzt. Dieser Ansatz 3 besitzt eine zylindrische Bohrung 4, in die der Füllkörper 5 eingesetzt ist. An seinem kanülenseitigen Ende weist der Füllkörper einen Bund 6 auf, der sich an der Stirnseite des Ansatzes 3 abstützt. Hierdurch wird die ortsfeste Lage des Füllkörpers im Ansatz gewährleistet. In Fließrichtung der Spritzflüssigkeit übernimmt die Kanüle mit ihrem Aufsatzteil 10 die Halterung des Füllkörpers.

Der Füllkörper 5 besitzt am Umfang eine Rille 7 für den Durchlaß einer medikamentösen Flüssigkeit. Auf den Ansatz 3 wird der Aufsatzteil 10 der Kanüle 8 aufgesetzt. Anstelle der Rille 7 kann auch eine mittige Bohrung im Füllkörper 5 vorgesehen sein.

Durch diese Ausbildungsform der Spritze verbleibt kein Restvolumen in der Bohrung des Ansatzes, weil der Füllkörper 5 bündig mit dem Spritzenzylinderboden 2 abschließt. Lediglich in der Kanüle verbleibt ein geringes Restvolumen. Die Kanüle mit ihrem Aufsatzteil 10 kann auch so verdreht werden, daß die Kanülenspitze beim Spritzen die richtige Lage einnimmt und zugleich auch die Stricheinteilung auf dem Spritzenzylinder sichtbar ist.

- Ansprüche -

846/10592

Firma ALMo Erzeugnisse Erwin Busch GmbH
Große Allee 84, 3548 Arolsen


A n s p r ü c h e
-------------------

1. Injektionsspritze, insbesondere eine Einmalinjektionsspritze, bestehend aus einem Spritzenzylinder, sowie einem an dem Zylinderboden angesetzten Ansatz, welcher die Kanüle trägt,
d a d u r c h   g e k e n n z e i c h n e t, daß
der Ansatz (3) einen Füllkörper (5) aufweist,
der mit einem Durchlaß für die medikamentöse
Flüssigkeit versehen ist.

2. Injektionsspritze nach Anspruch 1
d a d u r c h   g e k e n n z e i c h n e t , daß
der Füllkörpter (5) auf seinem Umfang als Durchlaß mindestens eine Rille 7 aufweist.

3. Injektionsspritze nach Anspruch 1
d a d u r c h   g e k e n n z e i c h n e t, daß
der Füllkörper (5) als Durchlaß mittig eine
durchgehende Bohrung (4) aufweist.

4. Injektionsspritze nach Anspruch 1
d a d u r c h   g e k e n n z e i c h n e t, daß
der Füllkörper (5) an seinem Ende eine Verdickung aufweist, die vorzugsweise als Bund (6)
ausgebildet ist.

5. Injektionsspritze nach Anspruch 1
d a d u r c h   g e k e n n z e i c h n e t , daß
die Länge des Füllkörpers der Bohrungslänge des
Ansatzes entspricht.

0165542

Fig. 1

*Fig. 2*

0165542